# EUROPEAN PATENT APPLICATION

(11) **EP 4 289 935 A1**
(43) Date of publication of application: **13.12.2023**
(21) Application number: 23174076.2
(22) Date of filing: 17.05.2023
(51) Int. Cl.: C12N 1/14, A01G 18/00, E04B 1/74, E04B 5/16, E04B 5/43

(54) **PROCESS OF PRODUCING A BUILDING FLOOR**

(30) Priority: 20.05.2022 NL 2031943
(71) Applicant: Green Floor IP B.V., 3207 KP Spijkenisse (NL)
(72) Inventor: Van Driel, Roland, 7827 TB EMMEN (NL); Borra, Hans Antonius, 3707 TB ZEIST (NL)
(74) Representative: De Vries & Metman

(57) **Abstract**

Floor and process of producing a floor on a surface by applying a layer of a substrate material inoculated with a mycelial inoculum, allowing mycelium to grow and colonize the substrate material to form the floor in-situ. A heat exchanger may be embedded in the substrate material, forming a reinforcement after the floor is completed.

## Description

The invention relates to a process of producing a building floor, and to a building floor produced with such a process. Hitherto such building floors are made by pouring concrete, e.g., in a formwork. The use of concrete is detrimental for the environment in several aspects. Pouring concrete contributes to CO2 emission. After use, recycling is difficult. The quality of the concrete may deteriorate due to leaching. Additional provisions are required to improve isolation.

The object of the invention is to provide a floor which is less damaging for the environment.

The object of the invention is achieved with a process of producing a building floor on a surface by applying a layer of a substrate material inoculated with a mycelial inoculum, allowing mycelium to grow and colonize the substrate material to form the building floor. This process can be carried out in-situ and has a substantially lower CO2 emission compared to pouring a concrete floor of the same size. The mycelial floor material can easily be recycled and the material is not leached or washed out.

The use of mycelium based material as a building material is disclosed in, e.g., WO 2008/073489 and WO 2021/180948. These references relate to a panels and similar parts moulded in a formwork. Production of a floor, e.g., in-situ by pouring inoculated substrate material, is not disclosed or suggested.

In a particular embodiment, first a cavity is formed in the surface, e.g., a ground surface, and the layer of substrate material is applied by pouring the inoculated substrate material into the cavity. The cavity defines the contour and the thickness of the floor. The cavity can for example be about 20 - 50 cm deep. The cavity can be stabilized, e.g., by compacting the soil. The bottom surface of the cavity can be leveled and equalized to ensure an even thickness of the floor.

The substrate material typically is an aqueous slurry with a high moisture content. To prevent leakage of water and dehydration of the substrate material, the surface of the cavity can first be covered with a water tight foil before the layer of inoculated substrate material is applied on the water tight foil.

The layer of inoculated substrate material can be covered with a vapor-sealing foil, which is removed after the substrate material is colonized by the mycelium. Such foils help to prevent the substrate material from drying out, but allow gas exchange and respiration processes necessary for mycelial growth.

To stimulate mycelial colonization the layer of inoculated substrate material can be heated to a temperature of, e.g., 18 - 24°C. The colonization step can for example take about 48 - 100 hours. Optionally, this can be reduced, e.g., by using electric growth stimulation. After removal of the vapor sealing foil the substrate material can be heated to permanently deactivate the mycelium and inhibit further growth of the mycelium, e.g., at a temperature of at least 45°C, e.g., for 24 hours or more, e.g., about 40 - 50 hours. The drying step can be reduced, e.g., by aeration.

The substrate material can for example be heated by at least one heating element in the cavity. The heating element is embedded in the substrate material and in a later stage in the produced building floor. The heating element can for example comprise a heat exchanger, e.g., with a channel connected to a source of a fluid heating medium, such as hot water. After the permanent deactivation of the mycelium, the heat exchanger is disconnected from the source. The heat exchanger remains in place within the produced floor, serving as a reinforcement. Additionally, or alternatively, other types of reinforcement can be applied.

The building floor can be finished and leveled with a top layer, e.g., of a leveling compound of a cementitious material or a concrete material.

Good results are obtained with mycelium selected from the following strains: Ganoderma Lucidem, Ganoderma lipsiense, Ganoderma adspersum, Pleurotus ostreatus, Pleurotus eryngii, Schizophyllum commune, Picnoporus sanguineus, Stropharia rugosoannulata and Phanerochaete chrysosporium. Other strains can also be used.

The substrate material can for example be based on biomass and/or saw dust. Optionally, fillers can be used, e.g., to increase or decrease specific weight of the substrate. Suitable fillers include perlite, vermiculite, lava, foam glass, bamboo shoots, pumice and the like. Other additives can also be used, for example for reinforcing the final floor.

The process results in a floor comprising at least one layer of an in situ colonized mycelium based material, e.g., comprising a heat exchanger embedded in the mycelium based material. Floors can be obtained with a floor insulation R-value above 3 m²K/W, e.g., above 3,5 m²K/W with a thickness of 150 mm, and/or having a compressive strength well above 300 kPa.

The invention is further explained with reference to the accompanying drawings schematically showing consecutive steps of an exemplary process according to the invention.
Figure 1: shows the step of providing a stabilized cavity with a watertight foil;
Figure 2: shows a next step providing a heat exchanger;
Figure 3: shows the step of vapour-sealing the inoculated substrate layer.

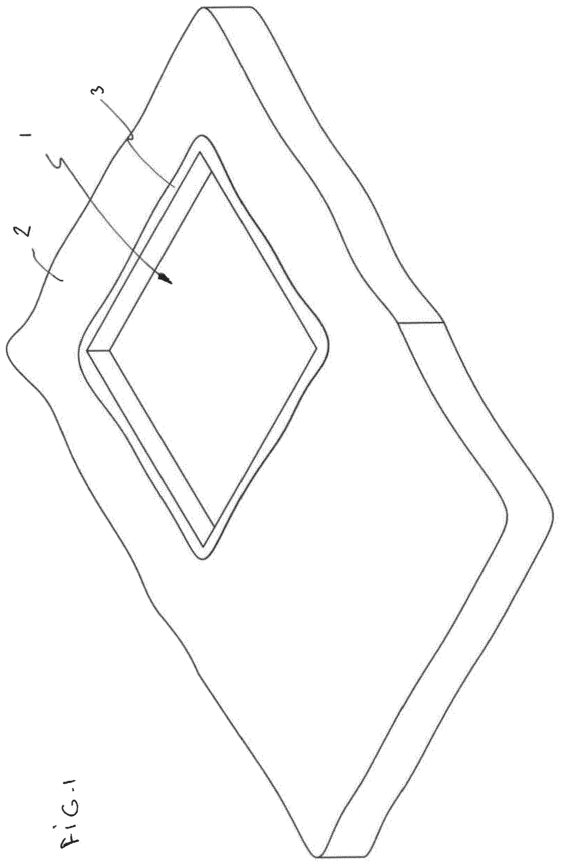

Figure 1 shows a rectangular cavity 1 in a soil or ground surface 2, e.g., of about 30 cm deep. The cavity 2 is stabilized and the bottom surface is equalized. The surfaces of the cavity 1 are covered with a watertight foil 3.

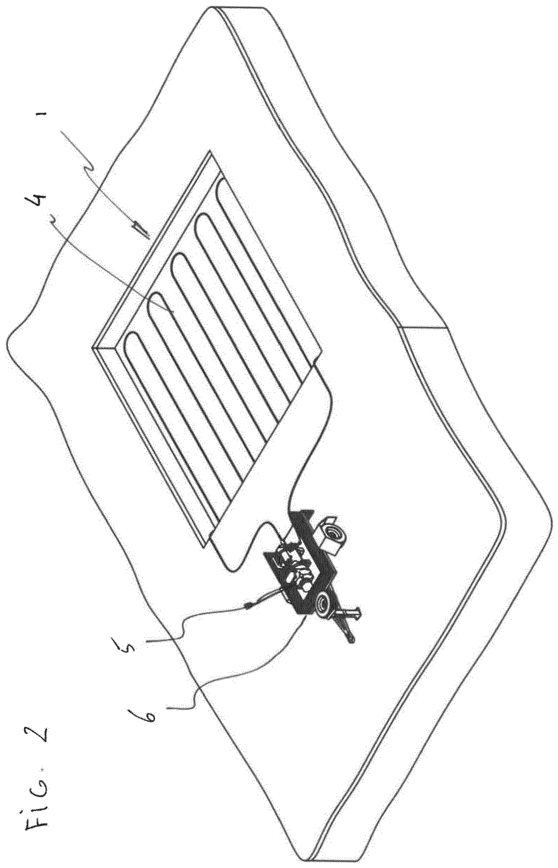

In a next step a heat exchanger 4 is deposited in the cavity 1, as shown in Figure 2. The heat exchanger 4 comprises a meandering tubular line defining a channel for a heating fluid, in particular water. The tubular line of the heat exchanger 4 is connected to a source 5 of heating fluid, in this case a mobile heat pump on a trailer 6.

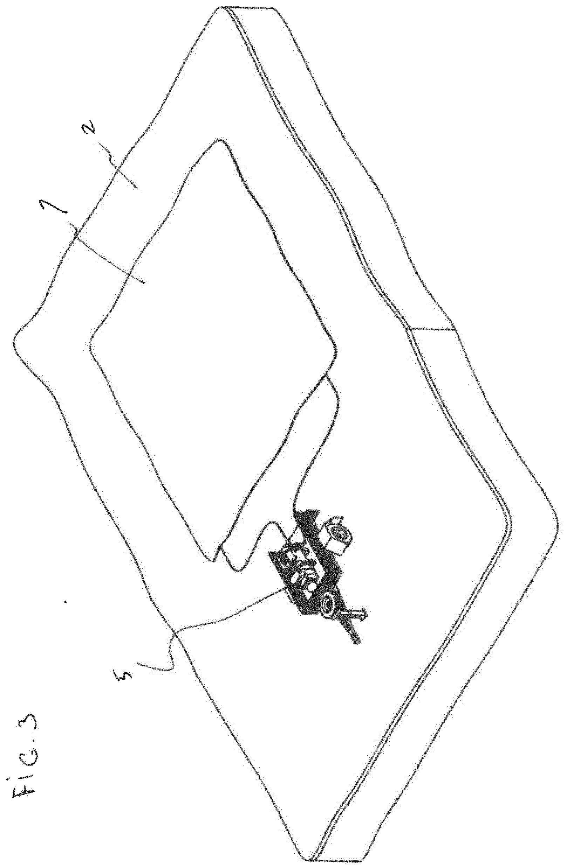

An inoculated substrate material, typically an aqueous slurry of biomass and/or sawdust, is poured into the cavity 1 on top of the watertight foil 3 embedding the heat exchanger 4. The watertight foil 3 prevents leakage of water from the slurry to the ground below. The layer of inoculated substrate material is then covered with a vapor-sealing foil 7, as shown in Figure 3. The watertight foil 3 below the substrate material, and the vapour-sealing foil 7 on top of the substrate material layer prevent dehydration of the substrate material in order to optimize conditions for mycelial colonization of the substrate material.

The heating pump 5 is then activated to pump heated water through the heat exchanger 4, so as to maintain the temperature of the substrate material at about 18 - 24°C. This stimulates growth of mycelium without formation of fruiting bodies.

Full colonization of the substrate by the mycelium usually takes about 48 - 100 hours. The degree of colonization can for example be checked by taking samples from the substrate. After full colonization of the substrate material, the vapour-sealing foil 7 is removed and the heat pump 5 is controlled to increase the temperature of the substrate material to at least 45°C for a period of at least about 48 hours. At these temperatures, the substrate material dehydrates and the mycelium is permanently inactivated.

In a next step, the heating is stopped and the heat exchanger 4 is disconnected from the heat pump 5 which is then removed from the site. The heat exchanger 4 itself remains embedded in the substrate material as a reinforcement. This results in a walkable reinforced building floor with a loading capacity well above 300 kPa.

The floor can then be finished, e.g., with a top layer of a self-leveling compound.

## Claims

1. Process of producing a building floor on a surface by applying a layer of a substrate material inoculated with a mycelial inoculum, allowing mycelium to grow and colonize the substrate material to form the building floor in-situ.

2. Process according to claim 1, wherein a cavity is formed in the surface and the layer of substrate material is applied by pouring the inoculated substrate material into the cavity.

3. Process according to claim 2, wherein the surface of the cavity is covered with a water tight foil and the layer of inoculated substrate material is applied on the water tight foil.

4. Process according to anyone of the preceding claims, wherein the layer of inoculated substrate material is covered with a vapor-sealing foil, which is removed after the substrate material is colonized by the mycelium.

5. Process according to any one of the preceding claims, wherein the layer of inoculated substrate material is heated to a temperature stimulating mycelial colonization, e.g., 18 - 24°C.

6. Process according to claim 4 and 5, wherein after removal of the vapor sealing foil the substrate material is heated to permanently deactivate the mycelium, e.g., at a temperature of at least 45°C.

7. Process according to claim 5 or 6, wherein the substrate material is heated by at least one heating element in the cavity.

8. Process according to claim 7, wherein the heating element comprises a heat exchanger.

9. Process according to claim 8, wherein the heat exchanger comprises a channel connected to a source of a fluid heating medium, e.g., hot water.

10. Process according to claim 8, wherein the heat exchanger is disconnected from the source after the permanent deactivation of the mycelium.

11. Process according to any one of the preceding claims, wherein the building floor is provided with a top layer, e.g., of a cementitious or concrete material.

12. Floor comprising at least one layer of an in situ colonized mycelium based material.

13. Floor according to claim 12, comprising one or more heat exchangers embedded in the mycelium based material.
